(19) Europäisches Patentamt
European Patent Office
Office européen des brevets

(11) Publication number: 0 218 350 B1

# (12) EUROPEAN PATENT SPECIFICATION

(45) Date of publication of patent specification: 10.04.91 Bulletin 91/15

(51) Int. Cl.$^5$: C07C 213/08, C07C 215/76

(21) Application number: 86306588.4

(22) Date of filing: 26.08.86

## (54) Process for preparing N-alkylaminophenols.

(30) Priority: 27.08.85 JP 187679/85
27.08.85 JP 187680/85

(43) Date of publication of application: 15.04.87 Bulletin 87/16

(45) Publication of the grant of the patent: 10.04.91 Bulletin 91/15

(84) Designated Contracting States: DE FR GB IT

(56) References cited:
DE-A- 3 402 997
US-A- 1 555 452
US-A- 2 150 832
CHEMICAL ABSTRACTS, vol. 92, no. 7, 18th February 1980, page 638, abstract no. 58415e, Columbus, Ohio, US & JP-A-79 55525

(73) Proprietor: SUMITOMO CHEMICAL COMPANY, LIMITED
Kitahama 4-chome 5-33
Chuo-ku Osaka 541 (JP)

(72) Inventor: Harada, Haruhisa
7-23-5, Aobadai
Ichihara-shi Chiba (JP)
Inventor: Maki, Hiroshi
1353-4, Shiizu
Ichihara-shi Chiba (JP)
Inventor: Sasaki, Shigeru
1-9, Yushudai Nishi
Ichihara-shi Chiba (JP)

(74) Representative: Diamond, Bryan Clive et al
Gee & Co., Chancery House, Chancery Lane
London WC2A 1QU (GB)

EP 0 218 350 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to an improved process for preparing N-alkylaminophenols. More particularly, this invention relates to a process for preparing an N-alkylaminophenol represented by the formula (III) :

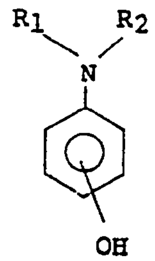

(III)

wherein $R_1$ represents a hydrogen atom or an alkyl group having from 1 to 6 carbon atoms ; and $R_2$ represents an alkyl group having from 1 to 6 carbon atoms, which process comprises reacting an aminophenol represented by the formula (I) :

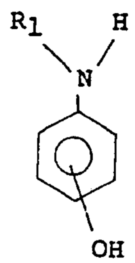

(I)

wherein $R_1$ is as defined above, with an alkyl halide represented by the formula (II) :

$$R_2X \quad (II)$$

wherein $R_2$ is as defined above, and X represents a halogen atom, in an autoclave in the presence of water under heat and pressure by using ammonia as an acid scavenger.

The N-alkylaminophenols represented by the formula (III) are well known to be extremely important compounds in the industry as intermediates of dyes for heat-sensitive or pressure-sensitive papers, xanthene dyes, fluorescent dyes, and the like.

Hitherto known processes for synthesizing the compounds represented by the formula (III) include a process in which sodium metanilate obtained from nitrobenzene is alkylated with an alkyl halide and the resulting product is subjected to alkali fusion, and a process comprising alkylating the compound represented by the formula (I) with the alkyl halide represented by the formula (II) using an alkali metal compound and/or an alkaline earth metal compound as an acid scavenger. The former process, the so-called alkali fusion process, produces a large quantity of waste water and a large quantity of a sludge and requires a long process and is, therefore, less attractive from the standpoint of industrial production. On the other hand, the latter process is superior to the former process as requiring one-step reaction. As described above, it is known to use alkali metal compounds, alkaline earth metal compounds, etc. as an acid scavenger. Specific examples thereof include sodium carbonate, sodium hydrogencarbonate, magnesium hydroxide, calcium hydroxide, etc. However, when the carbonates are used, carbonic acid gas produced fills the reaction vessel to considerably raise the reaction pressure, making it difficult to complete the reaction. Further, there is involved a disadvantage that undesired alkylation of the hydroxyl group of the formula (I) also proceeds under such a condition. On the other hand, use of the alkaline earth metal hydroxides is advantageous in that no carbonic acid gas is generated and alkylation of the hydroxyl group is suppressed. However, low water-solubility of the alkaline earth metal hydroxide not only makes stirring difficult but also makes it difficult to separate the produced N-alkylaminophenols from chlorides which are formed as by-products after completion of the reaction. Further, the amounts of these acid scavengers to be used greatly influence the selectivities of the N-alkylophenols. In order to obtain the highest

selectivity to the N-alkylaminophenols, the amount of the acid scavenger should be not more than an equivalent per alkyl group to be introduced. Therefore, the pH of the reaction system is always less than about 4, which forms a great problem of corrosion of the reaction vessel material.

In order to overcome these disadvantages, use of inorganic phosphates as an acid scavenger has been proposed as disclosed in Japanese Patent Aplication (OPI) No. 55525/79. (The term "OPI" as used herein refers to a published unexamined Japanese patent application). According to this process, however, the reaction rate from N-monoalkylaminophenols to N,N-dialkylaminophenols is low, and also phosphoric acid is released with the progress of the reaction to decrease the pH of the reaction system to 3 or less, which forms a serious problem for the material of the reaction vessel. Therefore, this process is far from being satisfactory.

US 2 150 832 discloses a method of making a tertiary amine by condensation of a primary aromatic amine and a hydrocarbon halide. A tertiary amine is added to the reaction process as a scavenger, to conbine with the hydrohalogenic acid which is formed during the reaction process and to leave the primary amine free for further reaction.

US 1 555 452 discloses a process of preparing isopropyl para-aminophenol by heating together isopropyl chloride and para-aminophenol. The process does not require the use of any scavenger during the reaction.

DE 3 402 997 discloses a process wherein 3-aminophenol is reacted with an aliphatic alcohol in an autoclave in the presence of phosphoric acid of an alkyl ester thereof as a catalyst.

This invention aims to eliminate the above-described disadvantages associated with the prior art, i.e., production of a large quantity of waste water or sludge, induction of side reactions, difficulty in separation of the product, influences on the material of the reaction vessel due to pH reduction, and the like.

A a result of extensive investigations in order to overcome these disavantages, it has now been found that the alkylation reaction can proceed very satisfactorily to thereby eliminate all the above-described disadvantages by carrying out the alkylation reaction in a water solvent under heat and pressure using ammonia as an acid scavenger. That is, the feature of the present invention lies in an advantage that the N-alkylaminophenols represented by the formula (III) can be prepared at low cost on an industrial scale by reacting the compound of the formula (I) and the compound of the formula (II) in a water solvent using ammonia as an acid scavenger. In addition, the starting compounds represented by the formulae (I) and (II) can be used as being wet without requiring drying. Hence, the present invention possesses great industrial values.

In the present invention, the compounds represented by the formula (I) specifically include aminophenols, N-methylaminophenols, N-ethylaminophenols, N-propylaminophenols, N-butylaminophenols, N-pentylaminophenols and N-hexylaminophenols. The alkyl halides represented by the formula (II) include, for example, methyl chloride, ethyl chloride, propyl chloride, butyl chloride, pentyl chloride, hexyl chloride, methyl bromide, ethyl bromide, propyl bromide, butyl bromide, pentyl bromide, hexyl bromide, methyl iodide, ethyl iodide, propyl iodide, butyl iodide, pentyl iodide and hexyl iodide.

The amount of these alkyl halides to be used is from 1 to 2 mols and preferably from 1 to 1.3 mols per alkyl group to be introduced. The amount of amonia, that is a characteristic of the present invention, is from 1 to 3 mols and preferably from 1.2 to 2.2 mols per mol of the compound represented by the formula (I).

In the present process, an alkali metal compound, an alkaline earth metal compound, an organic amine, etc. may be used in combination, if desired. The reaction temperature to be employed in the present invention is selected from 60° to 140°C and preferably from 80° to 120°C. The reaction rate is extremely low at a temperature lower than 60°C. On the other hand, at temperatures exceeding 140°C, hydrolysis of the alkyl halide and deterioration of the produed N-alkylaminophenols unfavorably become conspicuous.

The reaction is caried out under an elevated pressure of from 3 to 30 kg/cm$^2$G. If the reaction pressure is lower than 3 kg/cm$^2$G, the reaction rate is unsatisfactorily low. On the other hand, even when the reaction is carried out under a pressure exceeding 30 kg/cm$^2$G, there is no change in result as compared with that obtained under a pressure of from 3 to 30 kg/cm$^2$G and, therefore, there is no substantial merit in elevating the reaction pressure beyond the above-specified range. The reaction pressure may be maintained in the above-described range by either controlling the reaction pressure within the range of from 60° to 140°C or adjusting the charge ratio of the reactants. Further, the reaction pressure may also be maintained in the above-described range by supplementing a gas which is inert to the reaction, such as nitrogen or helium.

The reaction time varies depending upon the reaction temperature to be employed and the type of the compound of the formula (III) to be produced but is usually selected to be from 1 to 24 hours and preferably from 2 to 12 hours.

In the present invention, it is preferred that the ammonia is introduced to the reaction system in a continuous manner. In the case that the ammonia is continuously introduced to the reaction system, the pH of the reaction system is maintained at from about 4 to 10 and preferably from about 4 to 8.5. The time for introduction of the ammonia varies depending on the reaction temperature and is selected from, for example, 2 to 10 hours and preferably 2 to 6 hours at a reaction temperature of 100°C. The ammonia to be introduced may be in the form

of either aqueous ammonia or liquid ammonia. It is sufficient for water to be present in such an amount that makes stirring possible in the initial stage of the reaction. Specifically, the amount of water to be present is from 0.05 to 4 parts by weight and preferably from 0.15 to 2 parts by weight per part by weight of the aminophenol charged, in the initial stage of the reaction. If the amount of water to be present is lower than 0.05 part by weight, the temperature control of the reaction is not satisfactory, whereby occurrence of undesirable side reactions increases. On the other hand, if it exceeds 4 parts by weight, the volumetric efficiency is poor and undesired hydrolysis of the alkyl halide becomes conspicuous.

After completion of the continuous introduction of ammonia, it is preferred to perform an aging reaction for from 0 to 4 hours and preferably from 0 to 2 hours. In particular, in the case that dialkylaminophenols are produced from the aminophenols, the aging reaction is meaningful in a view point that conversion from the monoalkylaminophenols to the dialkylaminophenols is carried out sufficiently. However, an excessively long time for the aging reaction is meaningless because it merely makes the reaction time long and unfavorable formation of large quantities of impurities occurs.

The N-alkylation according to the present invention can be applied to introduction of either one or two alkyl groups to the compound represented by the formula (I). The reaction may be effected through one step or stepwise depending on the number or kind of the alkyl group to be introduced.

This invention will now be illustrated in greater detail with reference to the following examples, but it should be understood that the present invention is not limited thereto.

EXAMPLE 1

In a 200 cc-volume SUS-made autoclave equiped with a stirrer were charged 30.5 g (0.2795 mol) of m-aminophenol, 44,0 g (0.6822 mol) of ethyl chloride, 12.3 g of water and 21.8 g (0.3590 mol) of 28% aqueous ammonia to effect the reaction at 100°C for 6 hours. After completion of the reaction, the reaction mixture was rapidly separated into an oily phase and an aquous phase, each of which was then analyzed by gas chromatography and gel-permeation chromatography. As a result, m-aminophenol conversion was 99.7% ; N,N-diethyl-m-aminophenol selectivity was 82.0% ; N-ethyl-m-aminophenol selectivity was 12.8% ; m-diethylphenetidine selectivity was 1.1% ; and a dimer selectivity (see Note 1) was 0.5%. In the above reaction, the maximum reaction pressure was 11.0 $kg/cm^2G$, and the pH of the reaction mixture was 3.8.

(Note 1) : The selectivity to a dimer was obtained based on the estimated structural formula shown below (molecular weight : 229) :

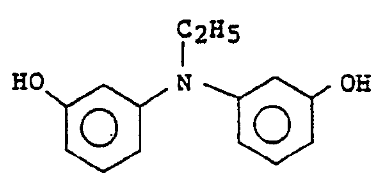

EXAMPLE 2

The reaction was carried out in the same manner as in Example 1 but using p-aminophenol in place of m-aminophenol. As a result of analyses of the reaction mixture in the same manner as in Example 1, p-aminophenol conversion was 99.8% ; N,N-diethyl-p-aminophenol selectivity was 81.2% ; N-ethyl-p-aminophenol selectivity was 12.6% ; p-diethylphenetidiene selectivity was 1.0% ; and dimer selectivity was 0.6%. The pH of the reaction mixture was 3.8.

EXAMPLE 3

The reaction was carried out in the same manner as in Example 1 but using ethyl bromide in place of ethyl chloride. As a result of analyses of the reaction mixture in the same manner as in Example 1, m-aminophenol conversion was 99.8% ; N,N-diethyl-m-aminophenol selectivity was 82.3% ; N-ethyl-m-aminophenol selectivity was 12.4% ; m-diethylphenetidine selectivity was 1.0% ; and dimer selectivity was 0.6%. The pH of the reaction mixture was 4.0

COMPARATIVE EXAMPLE 1

The reaction was carried out in the same manner as in Example 1 but using sodium hydrogenphosphate

(0.1795 mol) in place of aqueous ammonia. After completion of the reaction, the pH of the reaction mixture was as low as about 1.9, and the autoclave was found to suffer from slight corrosion. Analyses of the oily phase and the aqueous phase of the reaction mixture in the same manner as in Example 1 revealed that m-aminophenol conversion was 98.2% ; N,N-diethyl-m-aminophenol selectivity was 75.9% ; N-ethyl-m-aminophenol selectivity was 22.3% ; m-diethylphenetidine selectivity was 1.2% ; and dimer selectivity was 1.1%.

## COMPARATIVE EXAMPLE 2

The reaction was carried out in the same manner as in Example 1 but using diammonium hydrogenphosphate (0.1795 mol) in place of aqueous ammonia. The pH of the reaction mixture was as low as about 2.3, and the autoclave was found to suffer from slight corrosion. Analyses of the reaction mixture revealed that m-aminophenol conversion was 98.9% ; N,N-diethyl-m-aminophenol selectivity was 71.6% ; N-ethyl-m-aminophenol selectivity was 25.2% ; m-diethylphenetidine selectivity was 1.3% ; and dimer selectivity was 0.8%.

## COMPARATIVE EXAMPLE 3

The reaction was carried out in the same manner as in Example 1 but using sodium carbonate (0.1795 mol) in place of aqueous ammonia. The reaction pressure rose up to 32.6 kg/cm$^2$G. After completion of the reaction, the reaction mixture had a pH of 3.9. As a result of analyses of the reaction mixture, m-aminophenol conversion was 98.9% ; N,N-diethyl-m-aminophenol selectivity was 72.2% ; N-ethyl-m-aminophenol selectivity was 17.4% ; m-diethylphenetidine selectivity was 4.7% ; and dimer selectivity was 1.2%.

## COMPARATIVE EXAMPLE 4

The reaction was carried out in the same manner as in Example 1 but using magnesium hydroxide (0.1795 mol) in place of aqueous ammonia. In the initial stage of the reaction, stirring was almost impossible. After completion of the reaction, the reaction mixture had a pH of 3.7. Liquid seperation into an oily phase and an aqueous phase was impossible. After cooling, the oily content thus fixed was separated. Therefore, the fixed oily phase contained large amounts of inorganic salts formed as by-products. As reaction results, m-aminophenol conversion was 99.2% ; N,N-diethyl-m-aminophenol selectivity was 77.6% ; N-ethyl-m-aminophenol selectivity was 15.6% ; m-diethylphenetidine selectivity was 1.3% ; and dimer selectivity was 1.7%.

## EXAMPLE 4

In the same autoclave as used in Example 1 were charged 0.2795 mol of N-ethyl-m-aminophenol, 0.3411 mol of ethyl chloride, 12.3 g of water and 0.1795 mol of 28% aqueous ammonia, and the reaction was effected at 100°C for 4 hours. After completion of reaction, the reaction mixture was analyzed in the same manner as in Example 1. As a result, N-ethyl-m-aminophenol conversion was 99.3% ; N,N-diethyl-m-aminophenol selectivity was 97.0% ; m-diethylphenetidiene selectivity was 0.8% ; and dimer selectivity was 0.3%. The pH of the reaction mixture was 3.9.

## COMPARATIVE EXAMPLE 5

The reaction was carried out in the same manner as in Example 1 but using sodium carbonate (0.09 mol) in place of aqueous ammonia. The reaction pressure rose up to 31 kg/cm$^2$G. After completion of the reaction, the reaction mixture had a pH of 3.8. As a result of analyses of the reaction mixture, N-ethyl-m-aminophenol conversion was 96.2% ; N,N-diethyl-m-aminophenol selectivity was 89.8% ; m-diethylphenetidine selectivity was 4.8% ; and dimer selectivity was 1.0%.

## EXAMPLES 5 TO 7

The reaction was carried out in the same manner as in Example 1 with the exception shown in Table 1. The reaction results obtained are also shown in Table 1.

TABLE 1

| Example No. | Alkyl Chloride | m-Amiono-phenol Conversion (%) | N,N-Dialkyl Compound Selectivity (%) | N-Alkyl Compound Selectivity (%) | pH of Reaction Mixture |
|---|---|---|---|---|---|
| 5 | propyl chloride | 99.3 | 82.0 | 12.7 | 3.7 |
| 6 | butyl chloride | 98.9 | 80.5 | 13.5 | 3.8 |
| 7 | hexyl chloride | 98.8 | 81.0 | 13.2 | 3.8 |

EXAMPLE 8

Into a SUS-made 200 cc-volume autoclave equipped with a stirrer were charged 30.5 g (0.2795 mol) of m-aminophenol, 44.0 g (0.6822 mol) of ethyl chloride and 12.3 g of water. After the temperature of the mixture was elevated to 100°C, 515 g (0.606 mol) of 20% aqueous ammonia was continuously introduced to the reaction system over a period of time of 6 hours. After the prescribed amount of ammonia was introduced, the autoclave was rapidly cooled, and the reaction mixture was taken out, whereby the reaction mixture was promptly separated into an oily phase and an aqueous phase. Each of the thus separated liquids were analyzed by gas chromatography and gel-permeation chromathography. As a result, m-aminophenol conversion was 100% ; N,N-diethyl-m-aminophenol selectivity was 94.5% ; N-ethyl-m-aminophenol selectivity was 3.4% ; m-diethylphenetidine selectivity was 0.14% ; and dimer selectivity was 0.28%. The pH of the reaction mixture was 6.8.

EXAMPLE 9

The same procedure as in Example 8 was repeated except that the continuous introduction of 20% aqueous ammonia over 6 hours was followed by aging for 2 hours. The reaction results were as follows : m-aminophenol conversion = 100% ; N,N-diethyl-m-aminophenol selectivity = 95.1% ; N-ethyl-m-aminophenol selectivity = 1.4% ; m-diethyl-phenetidine selectivity = 0.29% ; dimer selectivity = 0.36%. The pH of the reaction mixture was 6.3.

EXAMPLE 10

The reaction was carried out in the same manner as in Example 8 but introducing 30.5 g (0.3588 mol) of 20% aqueous ammonia to the reaction system over a period of time of 6 hours. Analyses of the reaction mixture in the same manner as in Example 8 revealed that m-aminophenol conversion was 100% ; N,N-diethyl-m-aminophenol selectivity was 86.3% ; N-ethyl-m-aminophenol selectivity was 11.3% ; m-diethylphenetidine selectivity was 0% ; and dimer selectivity was 0.64%. The pH of the reaction mixture was 4.7.

EXAMPLE 11

The same procedure as in Example 8 was repeated except for continuously introducing 30.5 g (0.3588 mol) of 20% aqueous ammonia over a period of time of 4 hours followed by aging for 2 hours. The reaction results were as follows : m-aminophenol conversion = 100% ; N,N-diethyl-m-aminophenol selectivity = 94.8% ; N-ethyl-m-aminophenol selectivity = 3.6% ; m-diethylphenetidine selectivity = 0.25% ; and dimer selectivity = 0.21%. The pH of the reaction mixture was 4.7.

EXAMPLE 12

The reaction was carried out in the same manner as in Example 8 but using p-aminophenol in place of m-aminophenol to thereby obtain the following reaction results : p-aminophenol conversion = 100% ; N,N-diethyl-p-aminophenol selectivity = 94.8% ; N-ethyl-m-aminophenol selectivity = 3.2% ; p-diethylphenetidine selectivity = 0.13% ; dimer selectivity = 0.7%. The pH of the reaction mixture was 6.7.

EXAMPLES 13 TO 16

The reaction was carried out in the same manner as in Example 8 but replacing ethyl chloride with ethyl bromide (Example 13), propyl chloride (Example 14), butyl chloride (example 15) or hexyl chloride (Example 16). The results obtained are shown in Table 2.

TABLE 2

| Example No. | Alkyl Chloride | m-Amino-phenol Conversion (%) | N,N-Dialkyl Compound Selectivity (%) | N-Alkyl Compound Selectivity (%) | pH of Reaction Mixture |
|---|---|---|---|---|---|
| 13 | ethyl bromide | 100 | 94.9 | 3.1 | 6.9 |
| 14 | propyl chloride | 100 | 95.0 | 3.4 | 6.7 |
| 15 | butyl chloride | 100 | 93.8 | 3.7 | 6.6 |
| 16 | hexyl chloride | 100 | 93.1 | 4.0 | 6.7 |

EXAMPLE 17

In the same manner as in Example 8, N-ethyl-m-aminophenol (0.2795 mol) in place of of m-aminophenol, ethyl chloride (0.3411 mol) and 12.3 g of water were charged in the autoclave, and 10.9 g (0.1795 mol) of 28% aqueous ammonia was continuously introduced into the reaction system at 100°C over a period of time of 4 hours. After completion of the reaction, the reaction mixture was analyzed in the same manner as in Example 8. As a result, the reaction results were as follows : N-ethyl-m-aminophenol conversion = 100% ; N,N-diethyl-m-aminophenol selectivity = 99.2% ; m-diethylphenetidine selectivity = 0.3% ; and dimer selectivity= 0.2%. The pH of the reaction mixture was 4.9.

It is seen that by the invention, use of ammonia as an acid scavenger in the N-alkylation reaction is effective to improve selectivity to N-alkylation and to improve liquid separability after completion of the reaction. Further, in the case that the ammonia is continuously introduced to the reaction system, selectivity to N-alkylated compounds can be further improved. Moreover, the reaction results are not reduced at all even if the amount of added ammonia is more than an equivalent per alkyl group to be introduced. This makes it possible to maintain the pH of the reaction mixture at about 4 or more. Therefore, the present invention is also effective from the standpoint of prevention of corrosion of the reaction vessel material. In addition, use of inexpensive ammonia offers an advantage that N-alkylaminophenols can be prepared with an industrial superiority.

## Claims

1. A process for preparing an N-alkylaminophenol represented by the general formula (III) :

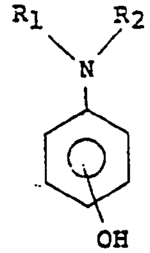

(III)

wherein $R_1$ represents a hydrogen atom or an alkyl group having from 1 to 6 carbon atoms ; and $R_2$ represents

an alkyl group having from 1 to 6 carbon atoms, which process comprises reacting an aminophenol represented by the formula (I) :

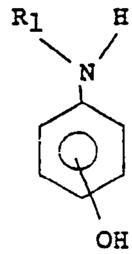

(I)

wherein $R_1$ is as defined above, with an alkyl halide represented by the formula (II) :

$$R_2X \quad (II)$$

wherein $R_2$ is as defined above and X represents a halogen atom, in an autoclave in the presence of water under heat and pressure by using ammonia as an acid scavenger.

2. A process for preparing an N-alkylaminophenol as claimed in Claim 1, wherein the amount of the alkyl halide represented by the formula (II) is from 1 to 2 mols per alkyl group to be introduced.

3. A process for preparing an N-alkylaminophenol as claimed in Claim 1 or 2, wherein the amount of ammonia is from 1 to 3 mols per mol of the aminophenol represented by the formula (I).

4. A process for preparing an N-alkylaminophenol as claimed in Claim 2 or 3, wherein the reaction is carried out at a temperature of from 60° to 140°C and a pressure of from 3 to 30 kg/cm² G for a period of time of from 1 to 24 hours.

5. A process for preparing an N-alkylaminophenol as claimed in any preceding claim, wherein the ammonia is continuously introduced to the autoclave as aqueous or liquid ammonia.

6. A process for preparing an N-alkylaminophenol as claimed in Claim 5, wherein the continuous introduction of ammonia is carried out while maintaining the pH of the reaction system at from 4 to 10.

7. A process for preparing an N-alkylaminophenol as claimed in Claim 5 or 6, wherein the ammonia is introduced over a period of time of from 2 to 10 hours at a temperature of 100°C.

8. A process for preparing an N-alkylaminophenol as claimed in Claim 5, 6 or 7, wherein the amount of water to be present is from 0.05 to 4 parts by weight per part by weight of the aminophenol represented by the formula (I).

9. A process for preparing an N-alkylaminophenol as claimed in Claim 5, 6, 7 or 8, wherein after completion of the continuous introduction of ammonia, an aging reaction is carried out.

10. A process for preparing an N-alkylaminophenol as claimed in Claim 9, wherein the aging reaction is carried out for from 0 to 4 hours.

## Ansprüche

1. Verfahren zur Herstellung eines N-Alkylaminophenols der allgemeinen Formel (III)

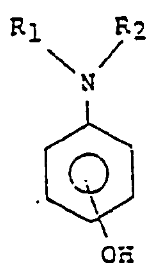

(III)

in der $R_1$ ein Wasserstoffatom oder einen Alkylrest mit 1 bis 6 Kohlenstoffatomen bedeutet, und $R_2$ einen Alkylrest mit 1 bis 6 Kohlenstoffatomen darstellt, umfassend die Umsetzung eines Aminophenols der Formel (I)

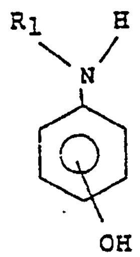

(I)

in der $R_1$ wie vorstehend definiert ist, mit einem Alkylhalogenid der Formel (II)

$R_2X$ (II)

in der $R_2$ wie vorstehend definiert ist, und X ein Halogenatom darstellt, in einem Autoklaven in Gegenwart vn Wasser unter Erhitzen und Druck und unter Verwendung von Ammoniak als Säureabfänger.

2. Verfahren zur Herstellung eines N-Alkylaminophenols nach Anspruch 1, wobei die Menge des Alkylhalogenids der Formel (II) 1 bis 2 Mol pro einzuführendem Alkylrest beträgt.

3. Verfahren zur Herstellung eines N-Alkylaminophenols nach Anspruch 1 oder 2, wobei die Menge an Ammoniak 1 bis 3 Mol pro Mol Aminophenol der Formel (I) beträgt.

4. Verfahren zur Herstellung eines N-Alkylaminophenols nach Anspruch 2 oder 3, wobei die Umsetzung bei einer Temperatur von 60 bis 140°C und einem Druck von 3 bis 30 $kg/cm^2$ G über einen Zeitraum von 1 bis 24 Stunden durchgeführt wird.

5. Verfahren zur Herstellung eines N-Alkylaminophenols nach einem der vorangehenden Ansprüche, wobei der Ammoniak kontinuierlich als wäßriger oder flüssiger Ammoniak in den Autoklaven eingespeist wird.

6. Verfahren zur Herstellung eines N-Alkylaminophenols nach Anspruch 5, wobei die kontinuierliche Einspeisung des Ammoniaks unter Aufrechterhaltung des pH-Wertes des Reaktionssystems auf 4 bis 10 durchgeführt wird.

7. Verfahren zur Herstellung eines N-Alkylaminophenols nach Anspruch 5 oder 6, wobei der Ammoniak in einem Zeitraum von 2 bis 10 Stunden bei einer Temperatur von 100°C eingespeist wird.

8. Verfahren zur Herstellung eines N-Alkylaminophenols nach Anspruch 5, 6 oder 7, wobei die Menge des vorhandenen Wassers 0,05 bis 4 Gewichtsteile pro Gewichtsteil des Aminophenols der Formel (I) beträgt.

9. Verfahren zur Herstellung eines N-Alkylaminophenols nach Anspruch 5, 6, 7 oder 8, wobei nach dem Abschluß der kontinuierlichen Einspeisung des Ammoniaks eine Alterungsreaktion durchgeführt wird.

10. Verfahren zur Herstellung eines N-Alkylaminophenols nach Anspruch 9, wobei die Alterungsreaktion 0 bis 4 Stunden durchgeführt wird.

**Revendications**

1. Un procédé de préparation d'un N-alkylaminophénol représenté par la formule générale (III) :

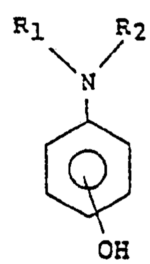

(III)

dans laquelle $R_1$ représente un atome d'hydrogène ou un groupe alkyle ayant de 1 à 6 atomes de carbone ; et $R_2$ représente un groupe alkyle ayant de 1 à 6 atomes de carbone, ledit procédé comprenant la réaction d'un aminophénol représenté par la formule (I) :

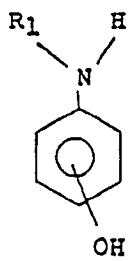

(I)

dans laquelle $R_1$ est comme défini ci-dessus, avec un halogénure d'alkyle représenté par la formule (II) :

$$R_2X \quad (II)$$

dans laquelle $R_2$ est comme défini ci-dessus et X représente un atome d'halogène, dans un autoclave en présence d'eau sous chauffage et pression en utilisant de l'ammoniaque comme accepteur d'acide.

2. Un procédé de préparation d'un N-alkylaminophénol selon la revendication 1, selon lequel la quantité d'halogénure d'alkyle représenté par la formule (II) est de 1 à 2 mol par groupe alkyle à introduire.

3. Un procédé de préparation d'un N-alkylaminophénol selon la revendication 1 ou 2, selon lequel la quantité d'ammoniaque est de 1 à 3 mol par mol d'aminophénol représenté par la formule (I).

4. Un procédé de préparation d'un N-alkylaminophénol selon la revendication 2 ou 3, selon lequel la réaction est conduite à une température de 60° à 140°C et à une pression de 3 à 30 kg/$cm^2$ manométrique pendant une période de temps de 1 à 24 h.

5. Un procédé de préparation d'un N-alkylaminophénol selon l'une quelconque des revendications précédentes, selon lequel l'ammoniaque est introduite en continu dans l'autoclave sous la forme d'ammoniaque aqueuse ou d'ammoniaque liquide.

6. Un procédé de préparation d'un N-alkylaminophénol selon la revendication 5, selon lequel l'introduction continue de l'ammoniaque est conduite tout en maintenant le pH du système réactionnel dans l'intervalle de 4 à 10.

7. Un procédé de préparation d'un N-alkylaminophénol selon la revendication 5 ou 6, selon lequel l'ammoniaque est introduite sur une période de temps de 2 à 10 h à une température de 100°C.

8. Un procédé de préparation d'un N-alkylaminophénol selon la revendication 5, 6 ou 7, selon lequel la quantité d'eau qui doit être présente est de 0,05 à 4 parties en poids par partie en poids de l'aminophénol représenté par la formule (I).

9. Un procédé de préparation d'un N-alkylaminophénol selon la revendication 5, 6, 7 ou 8, selon lequel après l'achèvement de l'introduction continue de l'ammoniaque, une réaction de vieillissement est conduite.

10. Un procédé de préparation d'un N-alkylaminophénol selon la revendication 9, selon lequel la réaction de vieillissement est conduite pendant un temps de 0 à 4 h.